# EUROPÄISCHE PATENTSCHRIFT

(11) **EP 0 778 318 B1**
(45) Veröffentlichungstag und Bekanntmachung des Hinweises auf die Patenterteilung: **31.07.2002**
(21) Anmeldenummer: 96118832.3
(22) Anmeldetag: 25.11.1996
(51) Int. Cl.: C09B 57/04, C07D 209/44, C07D 403/06, C07D 403/12, C09B 67/48

(54) **Benzimidazol-isoindoleninfarbstoffe**
Benzimidazol-isoindolenine dyes
Colorants à base de benzimidazole-isoindolenine

(30) Priorität: 06.12.1995 DE 19545464
(43) Veröffentlichungstag der Anmeldung: 11.06.1997
(73) Patentinhaber: DyStar Textilfarben GmbH & Co. Deutschland KG, 60318 Frankfurt am Main (DE)
(72) Erfinder: Lorenz, Manfred, Dr., 51061 Köln (DE); Wanken, Klaus-Wilfried, 51373 Leverkusen (DE)

(56) Entgegenhaltungen:
- EP-A- 0 141 325
- EP-A- 0 172 512
- EP-A- 0 684 289
- FR-A- 1 537 299
- FR-A- 2 307 008

## Beschreibung

Die Erfindung betrifft Benzimidazol-isoindoleninfarbstoffe, Verfahren zu ihrer Herstellung sowie ihre Verwendung zum Färben von hydrophoben synthetischen Materialien.

Aus DE-A 16 70 748 sind bereits Benzimidazol-isoindoleninfarbstoffe ähnlich denen der Formel (I) bekannt, die jedoch entweder anwendungstechnische oder verarbeitungstechnische Nachteile aufweisen. Unter anwendungstechnischen Nachteilen sind beispielsweise ein zu geringes Zieh- oder Aufbauvermögen beim Färben von Polyester oder eine schlechte Lichtechtheit, insbesondere Heißlichtechtheit, wie sie bei Verwendung damit gefärbter Textilien auf dem Automobilsektor gefordert sind, zu verstehen.

Derartige Nachteile besitzt insbesondere der aus Beispiel 1 aus DE-A 16 70 748 bekannte Farbstoff, der der Formel (I) entspricht, wenn R² = H, R¹ = C₂H₅ und A = N bedeuten würde.

Unter verarbeitungstechnischen Nachteilen werden insbesondere eine schlechte Dispergierbarkeit und Mahlbarkeit verstanden, wodurch die färberische Anwendung erheblich erschwert wird.

Derartige Nachteile weist beispielsweise der gemäß FR-A 1 537 299 (Beispiel 85) erhaltene Farbstoff auf, der der nachfolgenden Formel (I) entspricht, sofern A = Cyanmethylen, R¹ = n-Butyl und R² für Wasserstoff stehen würde.

Es wurden Benzimidazol-isoindolenine gefunden, die der Formel I oder deren tautomeren Formen entsprechen, 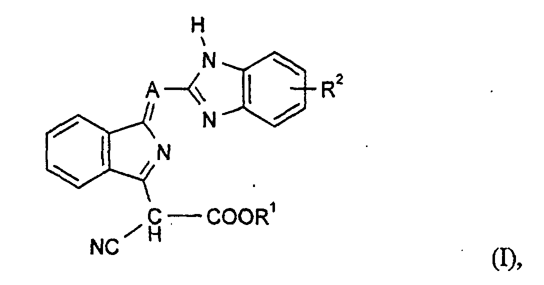 worin
- A: für N steht,
- R¹: für einen gesättigten oder ungesättigten, gegebenenfalls durch CN, oder Acyloxyrest, wie α-Acetoxyethyl, substituierten aliphatischen Rest mit 4 bis 20 C-Atomen, insbesondere mit 5 bis 8 C-Atomen, steht, oder
- A: für einen Cyanmethylenrest steht,
- R¹: für einen gesättigten oder ungesättigten, gegebenenfalls durch CN, gesättigten oder ungesättigten Oxyrest mit 1-4 C-Atomen, wie C₁-C₄-Alkoxy, Allyloxy und/oder ein Acyloxyrest, wie α-Acetoxyethyl, substituierten aliphatischen geradkettigen Rest mit 4 bis 20 C-Atomen, insbesondere mit 5 bis 8 C-Atomen, steht, der gegebenenfalls durch einen oder mehrere Sauerstoffatome unterbrochen ist, und
- R²: Wasserstoff, Halogen, insbesondere Cl, F und Br, C₁-C₄-Alkyl, ein gesättigter oder ungesättigter aliphatischer Oxyrest mit 1 bis 4 C-Atomen, insbesondere C₁-C₄-Alkoxy, der gegebenenfalls durch C₁-C₄-Alkoxy substituiert ist, CN oder NO₂ bedeutet, wobei R² ungleich Wasserstoff ist, wenn R¹ für n-Butyl steht.

Geeignete Reste R¹ sind z.B., n-Butyl, n-Pentyl, n-Hexyl, n-Heptyl, n-Octyl, n-Decyl, 2-Methoxy-ethyl, 2-Ethoxy-ethyl, 2-Butoxy-ethyl, 2-Allyloxy-ethyl, 2-Cyan-ethoxy-ethyl, 2-Cyanethoxy-butyl, 2-Acetoxy-ethyl, 3-Ethoxy-1-propyl, 2-Ethyl-hexyl. Als verzweigte Reste R¹ kommen vorzugsweise solche mit einer Methylseitenkette in Frage wie z.B. 1-Methoxy-2-propyl, 1-Ethoxy-2-propanyl, 3-Methoxy-butyl. Ganz besonders bevorzugt sind die Reste n-Butyl, n-Pentyl und 2-Butoxy-ethyl.

Bevorzugte Farbstoffe der Formel (I) sind solche, die der Formel (II) oder deren tautomeren Formen entsprechen 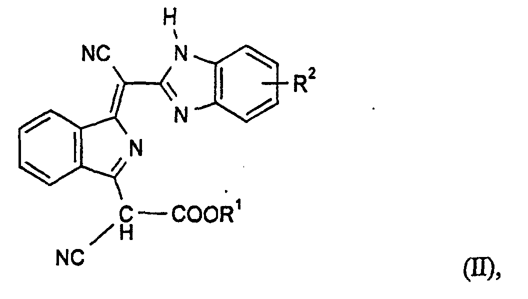 worin
- R¹: für einen vorzugsweise geradkettigen, gesättigten oder ungesättigten aliphatischen Rest mit 5 bis 8 C-Atomen steht, der gegebenenfalls substituiert und/oder durch ein oder mehrere Sauerstoffatome unterbrochen ist, und
- R²: die oben angegebene Bedeutung hat

Vorzugsweise steht
- R¹: für n-Amyl, n-Hexyl, n-Heptyl, n-Octyl, 2-Ethyl-hexyl, 2-Methoxy-ethyl, 2-Ethoxy-ethyl, 2-Butoxy-ethyl, 3-Methoxy-butyl, 1-Methyl-2-methoxy-ethyl und
- R²: für Wasserstoff oder Methyl.

Weiterhin bevorzugt sind Farbstoffe der Formel (I), die der Formel (III) oder deren tautomeren Formen entsprechen 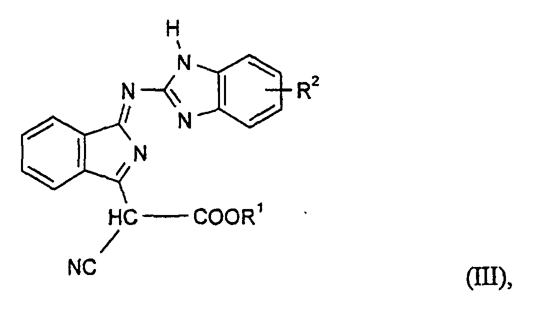 worin
R¹ *für einen gesättigten oder ungesättigten, gegebenenfalls durch CN, oder Acyloxyrest*, *wie α-Acetoxyethyl*, *substituierten aliphatischen Rest mit 4 bis 20 C-Atomen steht*, und R² die gleichen allgemeinen und bevorzugten Bedeutungen hat, wie sie für die Benzimidazolin-isoindolenine der Formel (II) angegeben sind., wobei R² ungleich Wasserstoff ist, wenn R¹ für n-Butyl steht.
Sämtliche in dieser Anmeldung beschriebenen Formeln stellen zwar lediglich eine, sofern mehrere denkbar sind, tautomere Form der jeweiligen Verbindung(en) dar, stehen aber stellvertretend für alle denkbaren tautomeren Formen.

Weiterhin umfaßt ein durch eine Formel beschriebenes E- oder Z-Isomer, insbesondere im Hinblick auf die exocyclische(n) Doppelbindung(en), auch jeweils das andere Isomer. Dies gilt, sofern nicht ausdrücklich etwas anderes gesagt wird.
Die Erfindung betrifft weiterhin ein Verfahren zur Herstellung von Verbindungen der Formel (I), das dadurch gekennzeichnet ist, daß man eine Verbindung der Formel (IV) 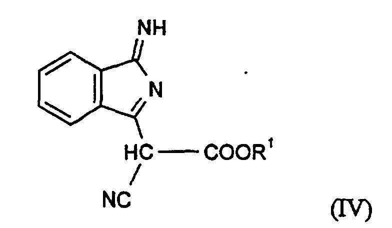 mit einem Benzimidazol der Formel (V) 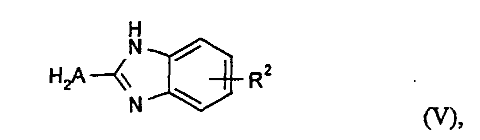 oder ein Aminoisoindolenin der Formel (VI) 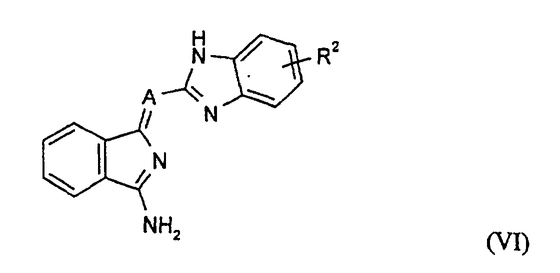 mit einem Cyanessigsäureester der Formel (VII)

NC-CH₂-COOR¹ (VII)

kondensiert, wobei
R¹, R² und A die oben angegebene Bedeutung besitzen.

In einer bevorzugten Ausführungsform erfolgt das erfindungsgemäße Verfahren zur Herstellung von Verbindungen der Formel (II)
a) durch Kondensation von Verbindungen der Formel (IV) mit Benzimidazolen der Formel (V), die der Formel (VIII) entsprechen 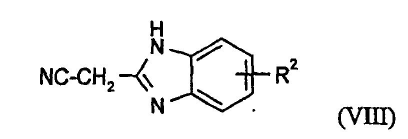 oder
b) durch Kondensation von Verbindungen der Formel (VI), die der Formel (IX) entsprechen 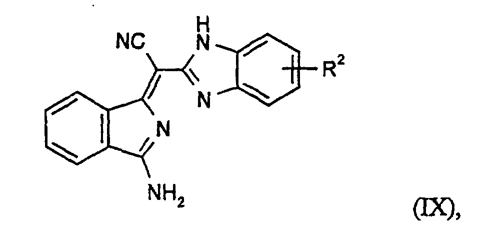 mit Cyanessigsäureester der Formel (VII),
worin die Substituenten R¹ und R² die oben angegebene Bedeutung haben, dadurch gekennzeichnet, daß das Verfahren in einem polaren, insbesondere hydrophilen, organischen Lösungsmittel durchgeführt wird.

Als polare Lösungsmittel sind beispielsweise zu nennen: Amide wie Dimethylformamid, Formamid, Dimethylacetamid, N-Methylpyrrolidon, ferner Dimethylsulfoxid, Acetonitril, Essigsäure oder Alkohol, wobei vorzugsweise der Alkohol eingesetzt wird, der auch in den Cyanessigsäureestern der Formel (VII) als alkoholische Komponente benutzt wird. Darüber hinaus können auch Mischungen dieser Lösungsmittel Verwendung finden.
Die Kondensation erfolgt im allgemeinen bei Temperaturen von 20 bis 150°C.
In einer besonders bevorzugten Ausführungsform dieses Verfahrens, welche auch für die Herstellung von Verbindungen der Formel (II) gelten soll, worin R¹ n-Butanol und R² Wasserstoff bedeutet, wird die Kondensation in Gegenwart einer organischen Säure durchgeführt.

Diese führt zu einer Beschleunigung der Reaktion, einer verbesserten Kristallinität und einer höheren Ausbeute. Als geeignete organische Säuren sind z.B. niedrige aliphatische, gesättigte oder ungesättigte Mono- oder Dicarbonsäuren wie z.B. Ameisensäure, Essigsäure, Propionsäure, Buttersäure, Oxalsäure, Fumarsäure, Maleinsäure, Bernsteinsäure, Glutarsäure, Adipinsäure, Äpfelsäure, Milchsäure, Zitronensäure, aber auch aromatische Säuren, wie beispielsweise Benzoesäure und Phthalsäure zu nennen. Die Säuren werden in Mengen von 0,2 bis 3 Mol-Äquivalenten, vorzugsweise 1 bis 2 Mol-Äquivalenten, jeweils bezogen auf das einzusetzende Ausgangsaminoisoindolin der Formel (IV) oder (IX) zugegeben. Aber auch höhere Mengen an Säure können eingesetzt werden bevorzugt dann, wenn die Säure gleichzeitig als Lösungsmittel dienen soll, wie beispielsweise Essigsäure.

Der Vorteil dieser bevorzugten Verfahrensweise liegt darin, daß Nebenprodukte, weitestgehend vermieden werden. Solche Nebenprodukte entsprechen insbesondere der Formel (X)

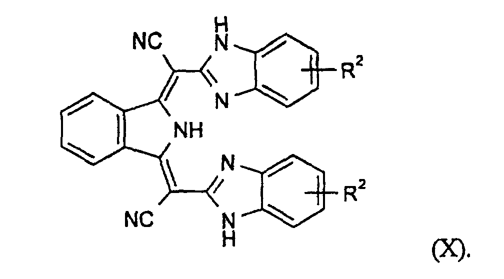

Sie sind rot und aufwendig aus dem Reaktionsgemisch zu entfernen. Der Farbton der gewünschten Farbstoffe wird daher durch einen substantiellen Anteil von (X) verändert. Dieser Nachteil kann beispielsweise bei dem in FR-A-1 537 299 in Beispiel 85 hergestellten Farbstoff beobachtet werden.
In einer besonders bevorzugten Ausführungsform des Verfahrens zur Herstellung von Verbindungen der Formel (II), worin R¹ auch für n-Butyl stehen kann, wenn R² Wasserstoff bedeutet, wird die Reaktion in Wasser oder einem wasserhaltigen Medium durchgeführt. Neben dem Wasser können dabei auch organische Lösungsmittel zugegen sein, und zwar bevorzugt solche, die mit Wasser ganz oder teilweise mischbar sind wie z.B. Alkohole, bevorzugt die Alkohole, die dem Rest R¹ zugrunde liegen, Ketone wie z.B. Aceton, Methylethylketon, Cyclohexanon, Ether wie Tetrahydrofuran und Dioxan, Dimethylformamid, N-Methylpyrrolidon und andere. Es können jedoch auch mit Wasser nicht mischbare Lösungsmittel dem wäßrigen Reaktionsmedium zugesetzt werden, um z.B. die Kristallinität zu verbessern und besondere Kristallformen zu erzielen. Die organischen Lösungsmittel können von Anfang an zugegen sein oder aber auch erst im Verlauf der Reaktion zugegeben werden. Auch hierbei kann ein Zusatz einer organischen Säure sich vorteilhaft auswirken und zu einer Beschleunigung der Reaktion, einer verbesserten Kristallinität sowie einer höherer Ausbeute führen.
Das Arbeiten mit Wasser bzw. wasserhaltigen Reaktionsmedien erleichtert die Isolierung der Farbstoffe und vermeidet die Aufarbeitung von größeren Mengen an organischen Lösungsmitteln. Bevorzugt wird bei dieser Verfahrensvariante mit Wassergehalten von 20 bis 100 %, insbesondere 50 bis 100 % gearbeitet (bezogen auf die Menge des verwendeten Reaktionsmediums). Diese Verfahrensweise ist insbesondere für den Weg a) ausgehend von Verbindungen der Formel VIII bevorzugt. Verwendet man Wasser oder ein überwiegend wäßriges Medium als Reaktionsmedium, so werden zweckmäßigerweise oberflächenaktive Substanzen wie Dispergiermittel, Emulgatoren und Netzmittel zugegeben. In Betracht kommen die bekannten nichtionogenen, anionischen und kationischen Hilfsmittel. Solche Verbindungen sind z.B. Salze von Alkylbenzolsulfonsäuren, Alkylphenolsulfonsäuren, Alkylnaphthalinsulfonsäuren, Kondensationsprodukte aus Phenolsulfonsäuren, Fomaldehyd und Harnstoff, Ligninsulfonate, Additionsprodukte von Ethylen- und Propylenoxid an Alkanole, Alkandiole, Phenole, Carbonsäuren, Amine, Carbonsäureamide und ihre Schwefelsäurehalbester, wobei auch Mischungen dieser Verbindungen eingesetzt werden können. Besonders bevorzugt sind jedoch Ligninsulfonate wie z.B. Kraftlignine vom Typ Reax der Firma Westvaco oder Sulfitlignine vom Typ Ufoxane der Firma Borregaard.

In einem ganz besonders bevorzugten Verfahren zur Herstellung von Verbindungen der Formel (II), worin R¹ auch für n-Butyl stehen kann, wenn R² Wasserstoff bedeutet, erfolgt die Kondensation entsprechend den Weg b) ausgehend von der Verbindung der Formel (IX) 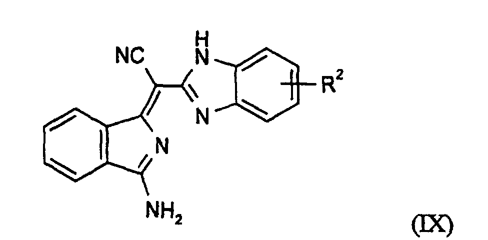 mit Cyanessigsäureester der Formel (VII), worin
R¹ und R² die oben angegebene Bedeutung haben, bei einer Temperatur von 100 bis 150°C, bevorzugt bei 110 bis 130°C, in einem polaren organischen Lösungsmittel, wie sie beispielsweise oben beschrieben sind, als Reaktionsmedium und in Gegenwart einer organischen Säure. Dabei werden die Ausgangskomponenten vorzugsweise in äquimolaren Mengen eingesetzt. Eine Komponente, vorzugsweise der Cyanessigsäureester der Formel (VII), kann aber auch in einem 5 bis 50 %igen, vorzugsweise 10 bis 30 %igen molaren Überschuß eingesetzt werden.

Auf diese Weise werden einerseits sehr kurze Reaktionszeiten und andererseits sehr reine Endprodukte realisiert.

Die oben geschilderten Verfahrensvarianten sind in der Lage, den Anteil an unerwünschten Nebenprodukten der Formel (X) deutlich unter 2 %, vorzugsweise deutlich unter 1 % zu halten.

Bevorzugt ist weiterhin eine Verfahrensvariante zur Herstellung von Farbstoffen der Formel (I), worin R¹ auch n-Butyl bedeuten kann, wenn R² für Wasserstoff steht, in der die Kondensation ausgehend von Verbindungen der Formel (IV) 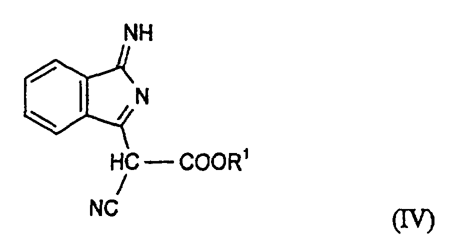 mit Benzimidazolen der Formel (VIII) 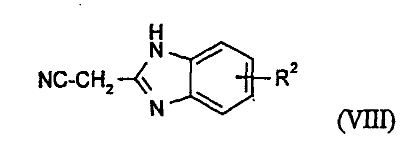 erfolgt, wobei die Kondensation vorzugsweise bei einer Temperatur von 25 bis 100°C durchgeführt wird.

Ebenfalls ganz besonders bevorzugt ist ein Verfahren zur Herstellung von Verbindungen der Formel (II), das dadurch gekennzeichnet ist, daß die eingesetzte Amineisoindolenin-Verbindung der Formel (IV) bzw. (IX) durch Kondensation von Amino-Imino-Isoindolenin der Formel (XI) 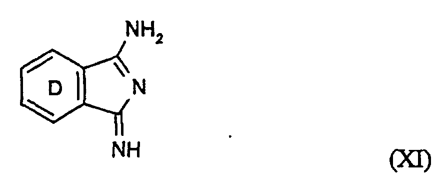 mit den Methinverbindungen der Formel (VII) bzw. (VIII) hergestellt wird. Dieses ganz besonders bevorzugte Verfahren läßt sich in dem folgenden Reaktionsschema darstellen:

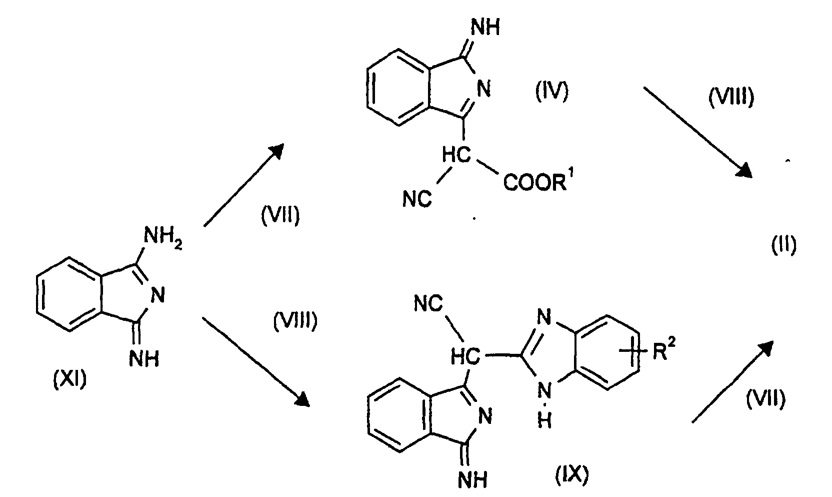

Bevorzugt ist demnach das obige Verfahren zur Herstellung von Farbstoffen der Formel (II), dadurch gekennzeichnet, daß die eingesetzte Verbindung der Formel (IV) durch Umsetzung der Amino-Imino-Isoindolin-Verbindung der Formel (XI) 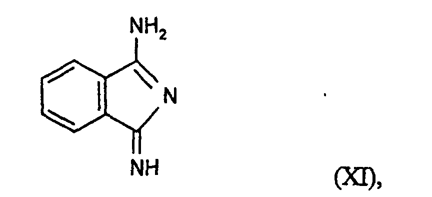 mit einem Cyanessigsäureester der Formel (VII)

NC-CH₂-COOR¹ (VII),

worin R¹ die obige Bedeutung hat,
erhalten wird, oder die eingesetzte Verbindung der Formel (IX) durch Umsetzung von Amino-Imino-Isoindolin der Formel (XI) mit einem 2-Cyanmethylbenzimidazol der Formel (VIII) 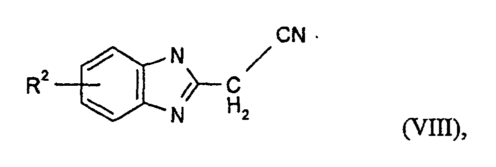 worin R² die obige Bedeutung hat, erhalten wird.

Diese erfindungsgemäße Verfahrenssequenz ausgehend von den Amino-Imino-Isoindolin-Verbindungen der Formel (XI) zu Farbstoffen der Formel (II) kann vorzugsweise ebenfalls in Wasser oder in einem wasserhaltigem Medium durchgeführt werden. Bei dieser Verfahrensweise ist insbesondere auch eine Eintopfreaktion möglich, d.h. ohne Zwischenisolierung der monokondensierten Verbindungen der Formel (VII) bzw. (IX).

Das Verfahren zur Herstellung von Verbindungen der Formel (III) ist dadurch gekennzeichnet, daß Verbindungen der Formel (VI), die der Formel (XII) entsprechen 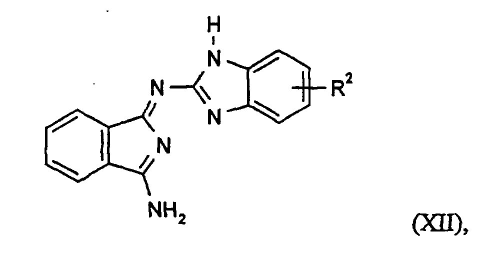 worin R² die obige Bedeutung hat,
mit Cyanessigsäureestern der Formel (VII)

NC-CH₂-COOR¹ (VII),

worin R¹ die obige Bedeutung hat,
kondensiert werden. Die bevorzugten Reaktionsbedingungen dieses Herstellungsverfahrens gelten auch für die Herstellung von Verbindungen der Formel (III), worin R ¹ für n-Butyl stehen kann, wenn R² für Wasserstoff steht und entsprechen denen für die Herstellung von Verbindungen der Formel (II). Auch hier wurde überraschenderweise gefunden, daß die Kondensationsreaktion unter sonst gleichen Bedingungen, besonders vorteilhaft in Wasser oder in Mischungen von Wasser mit organischen Lösungsmitteln durchgeführt werden kann. Bevorzugte Reaktionstemperaturen liegen bei 60-100°C.

Verbindungen der Formel (XII) können auf verschiedene Weise hergestellt werden. So lassen sich einerseits Amino-Imino-Isoindoleninen der Formel (XI) 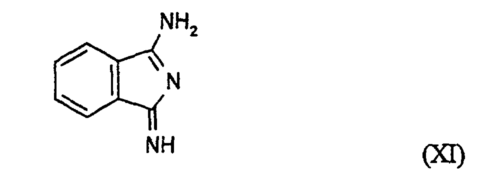 mit 2-Amino-benzimidazolen der Formel (V), die der Formel (XIII) entsprechen 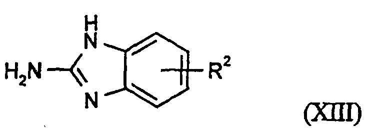 kondensieren, wobei R² die obige Bedeutung besitzt. Die Kondensation von (V) und (XI) zu (X) kann durch Erhitzen der Komponenten in einem organischen Lösungsmittel durchgeführt werden, wobei als Lösungsmittel beispielsweise Amide, wie Formamid, Dimethylformamid, Dimethylacetamid und N-Methylpyrrolidon und bevorzugt Alkohole, insbesondere niedere Alkohole wie Methanol, Ethanol, n-Propanol und iso-Propanol eingesetzt werden.

Ein weiteres Verfahren zur Herstellung von Verbindungen der Formel (XII) wurde gefunden, das dadurch gekennzeichnet ist, daß man 2-Aminobenzimidazole der Formel (XIII) 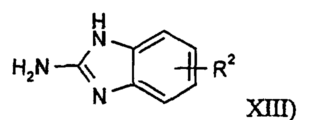 mit Phthalsäuredinitrilen der Formel (XIV) 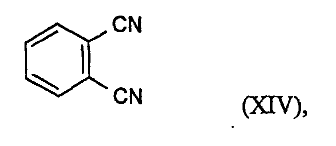 wobei R² die obige Bedeutung besitzt, in Gegenwart einer Base umsetzt.

Diese Additionsreaktion kann in einem organischen Lösungsmittel durchgeführt werden, wobei als Lösungsmittel Amide, wie beispielsweise Formamid, Dimethylformamid, Dimethylacetamid und N-Methylpyrrolidon und bevorzugt Alkohole und ganz besonders bevorzugt niedere Alkohole wie Methanol, Ethanol, n-Propanol und iso-Propanol verwendet werden können. Diese Reaktion wird vorzugsweise durch Alkoholate wie Natriummethylat, Natriumethylat, Kalium-tert.-Butylat katalysiert. Die Alkoholatmenge kann in weiten Grenzen variiert werden, bevorzugt sind jedoch Mengen zwischen 0,5 und 1 Mol-Äquivalent, bezogen auf die eingesetzte Menge an (XIV).
Geeignete Reaktionstemperaturen liegen zwischen 0 und 100°C, bevorzugt zwischen 20 und 60°C. Der Vorteil dieses Verfahrens zur Herstellung von Verbindungen der Formel (XII) ist der, daß das Aminoiminoisoindolenin, das normalerweise ebenfalls aus Phthalsäuredinitril hergestellt wird, nicht zuvor hergestellt werden muß. Somit werden höhere Ausbeuten, insbesondere höhere Raumzeitausbeuten erzielt, zumal diese Reaktion sehr schnell abläuft.
Zur Überwindung der verarbeitungstechnischen Nachteile der in FR-A 1 537 299, Beispiel 85 erhaltenen Verbindung der Formel (XV) 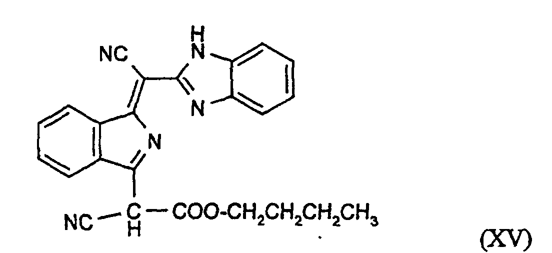 wurden überraschenderweise neue Kristallmodifikationen von (XV) gefunden, die die oben beschriebenen Nachteile nun nicht mehr besitzen.

In den aus FR-A-1 537 299 zur Herstellung von (XV) bekannten Verfahren, erfolgt die Kondensation ausgehend von der Verbindung der Formel (XVI) 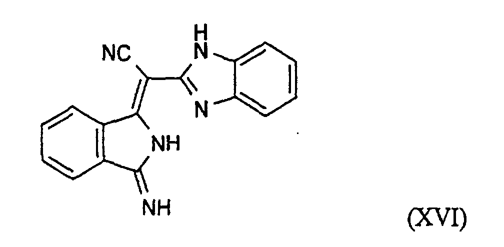 mit Cyanessigsäure-n-butylester (XVII) in Nitrobenzol bei einer Temperatur von 180°C. Dabei entsteht die β-Modifikation, die durch folgende mittlere bis starke Reflexionen im Röntgen-Pulverdiagramm gekennzeichnet ist, wobei die Daten den Netzebenenabstand in A und die relativen Intensitäten der Reflexionen in Prozent bedeuten (in Klammern). Für die Diffraktogramme, aufgenommen mit Cu-K_{α}-Strahlung, wurde ein rechnergesteuertes Siemens D-500 Pulverdiffraktometer verwendet.
β-Modifikation: 5,548 (70); 7,122 (100); 8,358 (70); 17,408 (91); 20,691 (53); 24,614 (54); 25,20 (55); 27,582 (65)
Die β-Modifikation kann auch erhalten werden, wenn die Kondensation von (XVI) mit (XVII) in n-Butanol erfolgt Sie fällt typischerweise in Form von fadenförmigen Kristallen an, die sich nur aufwendig formieren lassen.

Überraschenderweise wurden weitere Kristallmodifikationen (α-, γ- und ε) von (XV) gefunden, die sich sehr viel besser formieren lassen und somit überraschende verarbeitungstechnische Vorteile aufweisen.
Diese Modifikationen sind durch mittlere bis starke Reflexionen im Röntgenbeugungsdiagramm durch folgende Netzebenabstände in A mit den dazugehörigen relativen Intensitäten in Prozent (in Klammern) gekennzeichnet.
α-Modifikation: 9,298 (100); 19,300 (12); 23,080 (14); 24,199 (20); 24,574 (36); 26,457 (85); 27,795 (23); 28,656 (16)
γ-Modifikation: 5,007 (40); 5,474(87); 6,316 (45,2); 8,393 (52); 10,045 (53); 16,646 (45); 25,772 (62); 26,377 (100)
ε-Modifikation: 5,034 (50); 6,113 (100); 7,070 (29); 7,328 (25); 10,603 (33); 12,263 (23); 12,767 (21); 24,889 (24); 26,263 (30); 26,682 (23).

Die Erfindung betrifft weiterhin ein Verfahren zur Herstellung der Verbindung der Formel (XV) in seiner γ-Modifikation, dadurch gekennzeichnet, daß man die Kondensation von (XVI) und (XVII) in Dimethylformamid oder einem anderen polaren Lösungsmittel wie N-Methylpyrrolidon oder Dimethylsulfoxid bei einer Temperatur von 60 bis 140°C durchführt, oder das in der α-Modifikation vorliegende Produkt in einem solchen Lösungsmittel erhitzt oder daraus umkristallisiert, wobei die Kristallisation typischerweise bei Temperaturen unterhalb von 80°C, bevorzugt unterhalb von 60°C, erfolgt
Die α-Modifikation ist beispielsweise dadurch erhältlich, daß man die Reaktion in n-Butanol in Gegenwart aliphatischer Carbonsäuren, insbesondere aliphatische Dicarbonsäuren, wie Glutarsäure, durchführt. Die α-Modifikation fällt in kompakten Kristallen an.
Das Verfahren zur Herstellung der ε-Modifikation der Verbindung (XV) ist dadurch gekennzeichnet, daß man die Kondensation ausgehend von der Verbindung der Formel (XVIII) 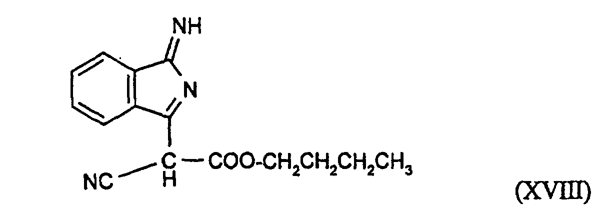 mit einer Verbindung der Formel (XIX) 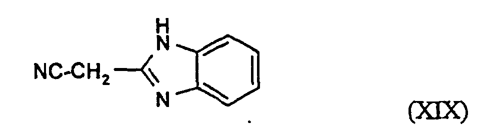 in einem wäßrigen Medium durchführt.

Bei Temperaturen von 60 bis 100°C entsteht die ε-Modifikation.

Dabei kann die Bildung dieser ε-Modifikation durch Tempern, sowie durch Zusatz eines organischen Lösungsmittels, bevorzugt n-Butanol, Zusatz von organischen Säuren wie z.B. Essigsäure oder Zusatz von Dispergiermitteln gefördert werden, wobei diese Maßnahmen einzeln oder in Kombination angewendet werden können.

Die Erfindung betrifft weiterhin die Verwendung von Farbstoffen der Formel (I) zum Färben von vollsynthetischen oder halbsynthetischen, hochmolekularen Stoffen. Besonders geeignet sind sie zum Färben oder. Bedrucken von synthetischen Fasermaterialien, insbesondere solchen aus aromatischen Polyestern und/oder Celluloseacetaten. Die dabei erhaltenen Färbungen besitzen eine hohe Farbstärke und zeigen eine überragende Lichtechtheit, insbesondere eine hohe Heißlichtechtheit und sind daher besonders zum Färben und Bedrucken von Textilmaterialien für die Automobilindustrie sowie für das Färben von sogenannten Microfasern geeignet.
Farbstoffe der Formel (I) eignen sich auch hervorragend für das sogenannte Thermo-Transfer-Printing auf textilen und nichttextilen Substraten z.B. nach dem D2T2 (Dye Diffusion Thermo Transfer)-Prozeß für die Bildaufzeichnung. Weiterhin können die Farbstoffe zum Massefärben von Kunststoffen zB. von Polyethylenen, Polypropylenen, Polystyrol, Polycarbonaten sowie von Kunststoffblends wie z.B. ABS verwendet werden. Die Farbstoffe fluoreszieren teilweise und eignen sich daher auch als Fluoreszenzfarbstoffe. Bevorzugte Fluoreszensfarbstoffe sind dabei Farbstoffe der Formel (II).

Textilmaterialien aus Polyester können mit den erfindungsgemäßen Farbstoffen nach Art einer Spinnfärbung gefärbt werden, bevorzugt jedoch aus wäßriger Suspension. Hierzu werden die Farbstoffe auf allgemein bekannte Weise zu Färbepräparaten verarbeitet, z.B. durch Mahlen in Wasser in Gegenwart von Dispergier- und/oder Füllmitteln. Mit den gegebenenfalls im Vakuum oder durch Zerstäuben getrockneten Präparaten kann man nach Zugabe von Wasser in sogenannter kurzer oder langer Flotte färben, klotzen oder bedrucken.

Zur Herstellung bzw. Verbesserung des Dispersionsgrades kann bei der Mahlung oder der Synthesereaktion ein oberflächenaktives Mittel oder ein Gemisch derartiger Hilfsmittel zugesetzt werden. Selbstverständlich kann die Teilchengröße der Farbstoffpartikel durch eine Mahlbehandlung z.B. Naßperlmahlung, sei es während der Synthese oder im Anschluß daran entsprechend beeinflußt und auf einen geforderten Wert eingestellt werden.
Als Dispergiermittel kommen solche anionischer oder nicht anionischer Natur in Betracht. Neben Dispergiermitteln der einen oder anderen Gruppe können auch Dispergiermittelgemische eingesetzt werden, wobei in erster Linie Gemische von nichtionischen und anionischen Dispergiermitteln gemeint sind, da anionische und kationische Dispergiermittel im Gemisch untereinander zur Bildung von Ausfällungen neigen.

Bei den anionischen Dispergiermitteln haben sich insbesondere Kondensationsprodukte von aromatischen Sulfonsäuren mit Formaldehyd, wie Kondensationsprodukte aus Formaldehyd und Alkylnaphthalinsulfonsäuren oder aus Formaldehyd, Naphthalinsulfonsäuren und Benzolsulfonsäure, Kondensationsprodukte aus gegebenenfalls substituiertem Phenol mit Formaldehyd und Natriumbisulfit als wirksam erwiesen.

Weiterhin kommen vor allem Ligninsulfonate in Betracht, z.B. solche, die nach dem Sulfit- oder Kraft-Verfahren gewonnen werden. Vorzugsweise handelt es sich um Produkte, die zum Teil hydrolysiert, oxidiert, propoxyliert oder desulfoniert und nach bekannten Verfahren fraktioniert werden, z.B. nach dem Molekulargewicht oder nach dem Sulfonierungsgrad. Auch Mischungen aus Sulfit- und Kraftligninsulfonaten sind gut wirksam.

Besonders geeignet sind Ligninsulfonate mit einem durchschnittlichen Molekulargewicht zwischen 1.000 und 100.000, einem Gehalt an aktivem Ligninsulfonat von mindestens 80 % und vorzugsweise mit niedrigem Gehalt an mehrwertigen Kationen.

Der Sulfonierungsgrad kann in weiten Grenzen variieren.

Nichtionische Dispergiermittel oder Emulgatoren sind z.B. Umsetzungsprodukte von Alkylenoxiden mit alkylierbaren Verbindungen, wie z.B. Fettalkoholen, Fettaminen, Fettsäuren, Phenolen, Alkylphenolen, Arylalkylphenolen, Carbonsäureamiden und Harzsäuren.

Hierbei handelt es sich z.B. um Ethylenoxid-Addukte aus der Klasse der Umsetzungsprodukte von Ethylenoxid mit:
a) gesättigten und/oder ungesättigten Fettalkoholen mit 6 bis 20 C-Atomen; oder
b) Alkylphenolen mit 4 bis 12 C-Atomen im Alkylrest, oder
c) gesättigten und/oder ungesättigten Fettaminen mit 14 bis 20 C-Atomen, oder
d) gesättigten und/oder ungesättigten Fettsäuren mit 14 bis 20 C-Atomen oder
e) hydrierte und/oder unhydrierte Harzsäuren.

Als Ethylenoxid-Addukte sind im einzelnen genannt:
a) Umsetzungsprodukte von gesättigten und/oder ungesättigten Fettalkoholen mit 6 bis 20 C-Atomen, mit 5 bis 30 mol Ethylenoxid,
b) Umsetzungsprodukte von Alkylphenolen mit 4 bis 12 C-Atomen mit 5 bis 20 mol Ethylenoxid,
c) Umsetzungsprodukte von gesättigten und/oder ungesättigten Fettsäsuren mit 14 bis 20 C-Atomen mit 5 bis 20 mol Ethylenoxid.

Weitere bevorzugte Dispergiermittel sind alkoxylierte Styrol-Phenol-Kondensationsprodukte, die gegebenenfalls im Gemisch mit ihren anorganischen Estern, die durch Umsetzung des alkoxylierten Styrol-Phenol-Kondensationsproduktes mit anorganischen Säuren, wie beispielsweise Amidosulfensäure, erhalten werden, eingesetzt werden.

Insbesondere zum Färben von Polyester eignen sich auch Mischungen von Farbstoffen der Formel (I), wodurch unter Umständen das Zieh- und Aufbauvermögen der Farbstoffe sowie ihre Dispergierbarkeit verbessert werden können.

Die neuen Farbstoffmischungen können nach verschiedenen Verfahren hergestellt werden:
1. durch Mischen der separat hergestellten und formierten Einzelfarbstoff komponenten,
2. durch gemeinsame Formierung der separat hergestellten Einzelkomponenten,
3. durch gemeinsame Synthese von Mischungen zweier oder mehrerer Farbstoffe der Formel (I) aus Mischungen unterschiedlicher Vorprodukte.

Die Mischung der Farbstoffe erfolgt zweckmäßigerweise in geeigneten Mühlen, beispielsweise Kugel- oder Sandmühlen. Getrennt formierte Einzelfarbstoffe können aber auch durch Einrühren in Färbeflotten gemischt werden.

Besonders geeignet sind Mischungen von Farbstoffen der Formeln (II) und/oder (III), die sich lediglich in dem Rest R¹ unterscheiden.

Die Farbstoffe eignen sich jedoch auch hervorragend zur Herstellung von Mischungen mit anderen Dispersionsfarbstoffen zur Erzeugung von Braun-, Grau- oder Grüntönen auf der Faser, weil sie die Lichtechtheit dieser Farbstoffe nicht beeinträchtigen.

Eine weitere bevorzugte Ausführungsform der vorliegenden Erfindung betrifft Mischungen von einem oder mehreren der Farbstoffe der Formeln (I) bis (III) mit einem oder mehreren Farbstoffen, wie sie üblicherweise zum Färben von Polyesterfasern oder Polyestertextilmaterialien für Automobilbezugsstoffe verwendet werden. Bei diesen Farbstoffen zum Färben von Automobilbezugsstoffen kann es sich insbesondere um Azo-, Disazo-, Anthrachinon-, Nitro-, Naphthalimid- und Terephthalimid-Farbstoffe handeln. Besonders bevorzugte Farbstoffe für derartige Mischungen sind z.B. die Colour-Index-Farbstoffe Yellow 23, 42, 51, 59, 65, 71, 86, 108, 122, 163, 182, 211, Orange 29, 30, 32, 41, 44, 45, 61, 73, Rot 60, 82, 86, 91, 92, 127, 134, 138, 159, 167, 191, 202, 258, 279, 284, 302, 323, Blau 27, 54, 56, 60, 73, 77, 79, 79:1, 87, 266, 333, 361, Violett 27, 28, 57 und 95, wobei sich die Gewichtsverhältnisse der Farbstoffmischungen nach der gewünschten Farbnuance richten.

### Beispiele

### Beispiel 1

234,7 g (1,5 mol) technisches Amino-imino-isoindolenin der Formel (V) (92,8 %ig) und 235,5 g Cyanmethylbenzimidazol der Formel (VI) mit R² = Wasserstoff wurden mit 3600 ml Methanol verrührt und 6 Stunden unter Rückfluß erhitzt. Die ausgefallene Substanz wurde bei Raumtemperatur abgesaugt und mit Methanol und Wasser gewaschen. Nach dem Trocknen erhielt man 363,5 g (84,9 % der Theorie) eines Produktes der Formel:

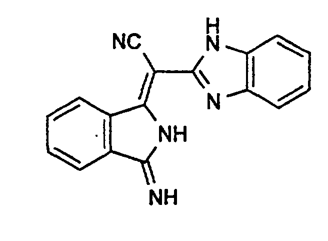

### Beispiel 2a

28,5 g einer gemäß Beispiel 1 hergestellten Substanz, 150 ml n-Butanol, 16,9 g Cyanessigsäurebutylester und 6 ml Eisessig wurden 10 Stunden unter Rückfluß erhitzt. Nach Abkühlen auf Raumtemperatur wurde die ausgefallene Substanz abgesaugt und mit Methanol und Wasser gewaschen. Nach dem Trocknen erhielt man 39,2 g eines Produktes, das nach HPLC 87,7 % eines gelben Farbstoffes der folgenden Formel in Form von langen filzigen Nadeln anfällt. Der Farbstoff färbte Polyesterfasern in gelben fluoreszierenden Tönen mit hervorragender Lichtechtheit.

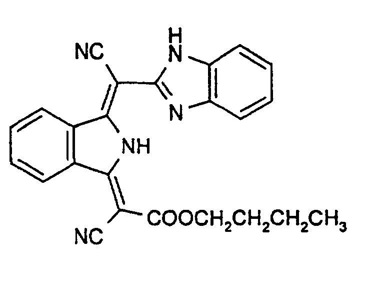

Die nach dem Verfahren erhältliche Substanz liegt in der β-Modifikation vor und wurde durch Umkristallisieren in Dimethylformamid bei 80°C gereinigt und lag danach in der γ-Modifikation vor. (Daten der jeweiligen Röntgenbeugungsmessungen siehe Beschreibung)

### Beispiel 2b

28,5 g einer gemäß Beispiel 1 hergestellten Substanz, 150 ml n-Butanol, 16,9 g Cyanessigsäurebutylester und 7 g Glutarsäure wurden 5 Stunden unter Rückfluß erhitzt. Nach Abkühlen auf Raumtemperatur wurde die ausgefallene Substanz abgesaugt und mit Methanol und Wasser gewaschen. Nach dem Trocknen erhielt man 39,2 g eines Produktes, das nach HPLC 87,7 % eines gelben Farbstoffes der folgenden Formel in Form von kompakten Kristallen anfällt. Der Farbstoff färbte Polyesterfasern in gelben fluoreszierenden Tönen mit hervorragender Lichtechtheit.

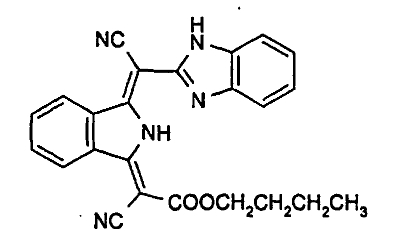

Die nach diesem Verfahren erhältliche Substanz liegt in der α-Modifikation vor. (Daten der jeweiligen Röntgenbeugungsmessungen siehe Beschreibung)

### Beispiel 4

28,5 g (0,1 mol) einer gemäß Beispiel 1 hergestellten Substanz, 150 ml N-Methylpyrrolidon, 16,9 g (0,12 mol) Cyanessigsäurebutylester und 6 ml Eisessig wurden 3 Stunden auf 120° erhitzt. Nach Abkühlen auf Raumtemperatur wurde 150 ml Wasser zugetropft, die ausgefallene Substanz abgesaugt und mit Methanol und Wasser gewaschen. Man erhielt 35,4 g der gleichen Substanz wie bei Beispiel 2. Der Farbstoff lag in der γ-Modifikation vor.

### Beispiel 5

36,8 g (0,24 mol) technisches Amino-imino-isoindolenin (92,8 %ig), 200 ml Wasser und 4,8 g Reax® 910 (ein mittelstark sulfoniertes Kraftlignin von Westvaco, USA), ein Dispergiermittel auf der Basis von Sulfolignin,wurden verrührt. Bei Raumtemperatur wurden in 10 Minuten 34,7 ml (0,24 mol) Cyanessigsäurebutylester zugetropft und 2 Stunden auf 30°C sowie jeweils 1 Stunde auf 40°, 60° und 70° erhitzt. Danach werden 12 ml Eisessig und in ca. 30 Minuten 34,9 g (0,2 mol) Cyanmethylbenzimidazol eingetragen und noch 1 Stunde auf 70° und 3 Stunden auf 90° erhitzt. Nach dem Absaugen und Trocknen erhielt man 88,9 g eines Präparates, das nach HPLC 70,1 % des gleichen Farbstoffes wie bei Beispiel 2 enthielt. Das Produkt liegt in der ε-Modifikation vor und ließ sich leicht durch Mahlung in einer Perlmühle zerkleinern und formieren.

### Formierungsbeispiel 1

26 g des in Beispiel 5 erhaltenen Farbstoffs (in Form des wasserfeuchten Preßkuchens) wurden in 200 ml Wasser mit 55 g Ligninsulfonat, Na-Salz und 5 g eines nichtionischen Dispergiermittels (Additionsprodukt aus Abietinsäure und 50 Moläquivalente Ethylenoxid) versetzt und mit Schwefelsäure auf pH-Wert 7 gestellt. Anschließend wurde 1 Stunde bei Raumtemperatur in der Perlmühle bis zur Feinverteilung (90 % < 1 µm) gemahlen, gesiebt und im Sprühtrockner getrocknet.

### Formierungsbeispiel 2

Es wurde wie in Formierungsbeispiel 1 verfahren, jedoch ersetzte man die 5 g nichtionisches Dispergiermittel durch 5 g eines Tensidgemisches auf Basis eines alkoxylierten Styrol-Phenol-Kondensationsproduktes (Phenol/Styrol = 2,8:1, 29 Moläquivalente Ethylenoxid) und deren anorganischen Ester (Amidosulfonsäure), das zusätzlich ein Kondensationsprodukt aus Ölsäure und 6,5 Moläquivalente Ethylenoxid enthält.

### Anwendungsbeispiel 1

2 g des nach Formierungsbeispiel 1 erhaltenen Pulvers wurden in 1 000 g Wasser dispergiert. Die Dispersion wurde mit 0,5 bis 2 g/l eines handelsüblichen Dispergiermittels auf Basis eines Kondensationsproduktes aus Naphthalinsulfonsäurenatriumsalz und Formaldehyd, 0,5 bis 2 g/l Mononatriumphosphat und 2 g eines handelsüblichen Egalisierhilfsmittels versetzt und mit Essigsäure auf einen pH-Wert von 4,5 bis 5,5 gestellt. In die so erhaltene Färbeflotte brachte man 100 g eines texturierten Polyestergewebes auf Basis Polyethylenglykolterephthalat ein und färbt 60 Minuten bei 130°C.

### Anwendungsbeispiel 2

In analoger Weise zu Beispiel 1 wurde auch das nach Formierbeispiel 2 erhaltene Farbstoffpräparat zum Färben verwendet.

Man erhielt farbstarke brillante Gelbfärbungen mit hervorragenden coloristischen Eigenschaften wie Lichtechtheit, wobei die thermische Stabilität der Farbstoffdispersion und die Egalität der Färbung aus Anwendungsbeispiel 2 noch verbessert ist

Mit vergleichbarer Ausbeute und Reinheit wurden die in Tabelle 1 aufgeführten Substanzen der Formel (II) erhalten, wenn man bei der Umsetzung wie in Beispiel 2 verfuhr, aber die analogen Cyanessigsäureester und als Lösungsmittel die den Estern zugrundeliegenden Alkohole einsetzte. Die Farbstoffe besitzen den gleichen Farbton.

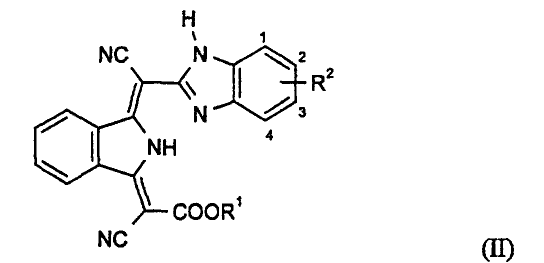

**Tabelle 1**

| Beispiel Nr. | R¹ | R²/Pos. | Farbton |
|---|---|---|---|
| Bsp. 6 | -CH₂CH₂CH₂CH₂CH₃ | H | fluorez. gelb |
| Bsp. 7 | -CH₂CH₂CH₂CH₂CH₂CH₃ | H | fluorsz. gelb |
| Bsp. 8 | -CH₂CH₂CH₂CH₂CH₂CH₂CH₃ | H | fluorsz. gelb |
| Bsp. 11 | -CH₂CH₂OCH₂CH₃ | H | fluorsz. gelb |
| Bsp. 12 | -CH₂CH₂OCH₂CH₂CH₂CH₃ | H | fluorsz. gelb |
| Bsp. 13 | -CH₂CH₂CH(CH₃)OCH₃ | H | fluorsz. gelb |
| Bsp. 14 | -CH₂CH₂CH₂CH₂CH₃ | -CH₃/3 | fluorsz. orange |
| Bsp. 16 | -CH₂CH₂CH₂CH₂CH₃ | -OCH₃/3 | fluorsz. gelbstichig scharlach |

### Beispiel 17

133,2 g (1 mol) 2-Amino-benzimidazol, 187,8 g (1,2 mol) technisches Amino-iminoisoindolenin und 400 ml Formamid wurden 10 Stunden auf 70°C erhitzt. Man ließ auf Raumtemperatur abkühlen, saugte ab und wusch mit Methanol. Nach dem Trocknen erhielt man 193,4 g (74 % der Theorie) eines Produktes der Formel:

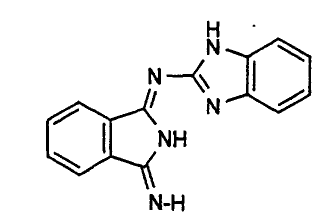

### Beispiel 18

26,1 g (0,1 mol) einer gemäß Beispiel 17 hergestellten Substanz und 150 ml n-Butanol wurden verrührt. Danach wurden 6 ml Eisessig und 17,3 ml (0,12 Mol) Cyanessigsäurepentylester zugegeben und 4 Stunden auf 90° erhitzt. Das ausgefallene Produkt wurde bei Raumtemperatur abgesaugt und mit Methanol gewaschen. Nach dem Trocknen erhielt man 33,3 g eines gelben Farbstoffes der folgenden Formel. Das Absorptionsmaximum in DMF liegt bei 419 nm.

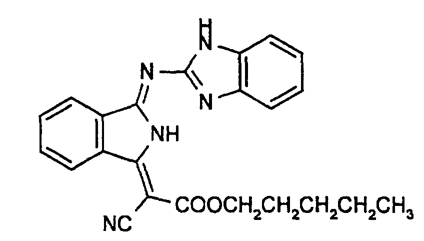

Der Farbstoff färbt Polyester in grünstichig gelben Tönen und zeigt eine hervorragende Lichtechtheit und eine hohe Farbstärke.
Mit vergleichbarer Ausbeute und Reinheit wurden die in Tabelle 2 aufgeführten Substanzen der Formel (XIV) erhalten, wenn man bei der Umsetzung wie bei Beispiel 17 verfuhr, aber die analogen Cyanessigsäureester und als Lösungsmittel die den Estern zugrundeliegenden Alkohole einsetzte. Die Farbstoffe besitzen den gleichen Farbton wie der Farbstoff von Beispiel 15.

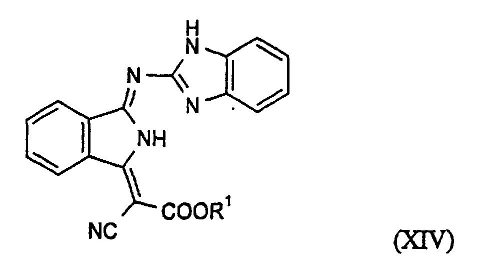

**Tabelle 2**

| Beispiel Nr. | R¹ |
|---|---|
| Bsp. 20 | -CH₂CH₂CH₂CH₂CH₂CH₃ |
| Bsp. 21 | -CH₂CH₂CH₂CH₂CH₂CH₂CH₃ |

## Patentansprüche

1. Verbindungen der Formel (I) 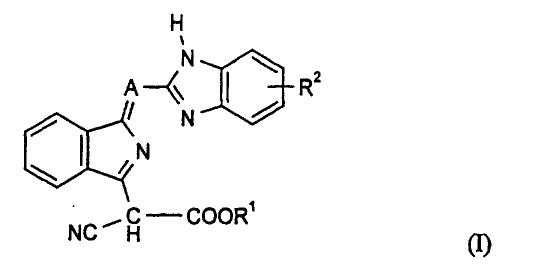 und deren tautomeren Formen, worin
A für N steht,
R¹ für einen gesättigten oder ungesättigten, gegebenenfalls durch CN, einen Acyloxyrest substituierten aliphatischen Rest mit 4 bis 20 C-Atomen, steht oder
A für einen Cyanmethylenrest steht und
R¹ für einen gesättigten oder ungesättigten, gegebenenfalls durch CN, gesättigten oder ungesättigten Oxyrest mit 1-4 C-Atomen, wie C₁-C₄-Alkoxy, Allyloxy und/oder ein Acyloxyrest substituierten aliphatischen geradkettigen Rest mit 4 bis 20 C-Atomen, steht, der gegebenenfalls durch einen oder mehrere Sauerstofatome unterbrochen ist und
R² Wasserstoff, Halogen, C₁-C₄-Alkyl, ein gesättigter oder ungesättigter aliphatischer Oxyrest mit 1 bis 4 C-Atomen, der gegebenenfalls durch C ₁-C₄-Alkoxy substituiert ist, CN oder NO₂ bedeutet, wobei R² ungleich Wasserstoff ist, wenn R¹ für n-Butyl steht.

2. Verbindungen gemäß Anspruch 1, die der Formel (II) 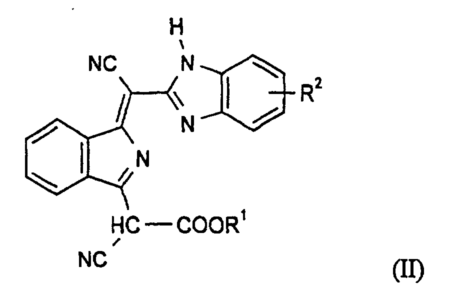 oder deren tautomeren Formen entsprechen, worin
R¹ für einen gesättigten oder ungesättigten, geradkettigen, gegebenenfalls durch CN, gesättigten oder ungesättigten Oxyrest mit 1-4 C-Atomen, wie C₁-C₄-Alkoxy, Allyloxy und/oder ein Acyloxyrest substituierten aliphatischen geradkettigen Rest mit 5 bis 8 C-Atomen steht, der gegebenenfalls durch einen oder mehrere Sauerstoffatome unterbrochen ist

3. Verbindungen gemäß Anspruch 1 der Formel (I), die der Formel (III) 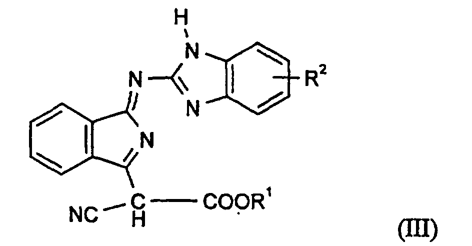 oder deren tautomeren Formen entsprechen, worin
R¹ für einen gesättigten oder ungesättigten, gegebenenfalls durch CN, einen Acyloxyrest substituierten aliphatischen Rest mit 4 bis 20 C-Atomen, steht.

4. Verfahren zur Herstellung von Verbindungen der Formel (I) gemäß Anspruch 1 oder deren tautomeren Formen, **dadurch gekennzeichnet, daß** man eine Verbindung der Formel (IV) 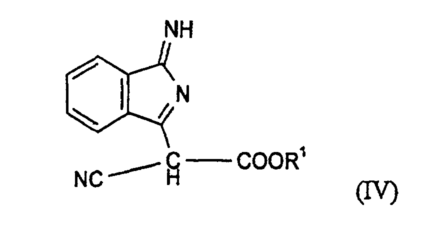 mit einem Benzimidazol der Formel (V) 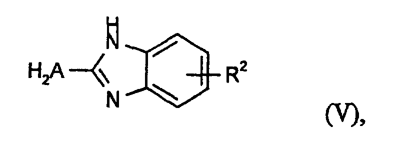 oder ein Aminoisoindolenin der Formel (VI) 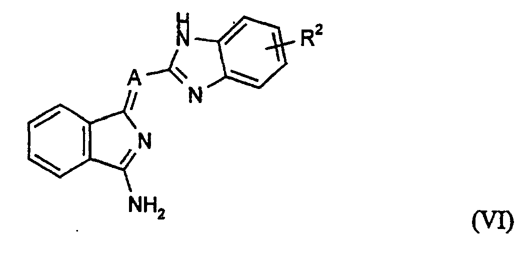 mit einem Cyanessigsäureester der Formel (VII)
NC-CH₂-COOR¹ (VII)
kondensiert, wobei
R¹, R² und A die in Anspruch 1 angegebene Bedeutung besitzen.

5. Verfahren gemäß Anspruch 4, **dadurch gekennzeichnet, daß**
A für einen Cyanmethylenrest steht und
R¹ für einen gesättigten oder ungesättigten, geradkettigen, gegebenenfalls durch CN, gesättigten oder ungesättigten Oxyrest mit 1-4 C-Atomen, wie C₁-C₄-Alkoxy, Allyloxy und/oder ein Acyloxyrest substituierten aliphatischen Rest mit 5 bis 8 C-Atomen steht, der gegebenenfalls durch einen oder mehrere Sauerstoffatome unterbrochen ist
R² Wasserstoff, Halogen, C₁-C₄-Alkyl, ein gesättigter oder ungesättigter aliphatischer Oxyrest mit 1 bis 4 C-Atomen, der gegebenenfalls durch C ₁-C₄-Alkoxy substituiert ist, CN oder NO₂ bedeutet..

6. Verfahren zur Herstellung von Verbindungen der Formel (I) oder deren tautomeren Formen 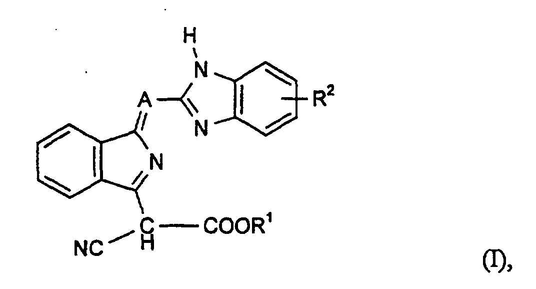 worin
A für N steht,
R¹ für einen gesättigten oder ungesättigten, gegebenenfalls durch CN, einen Acyloxyrest substituierten aliphatischen Rest mit 4 bis 20 C-Atomen, steht oder
A für einen Cyanmethylenrest steht und
R¹ für einen gesättigten oder ungesättigten, gegebenenfalls durch CN, oder gesättigten oder ungesättigten Oxyrest mit 1-4 C-Atomen, wie C₁-C₄-Alkoxy, Allyloxy und/oder ein Acyloxyrest substituierten aliphatischen geradkettigen Rest mit 4 bis 20 C-Atomen, steht, der gegebenenfalls durch einen oder mehrere Sauerstofatome unterbrochen ist und
R² Wasserstoff, Halogen, C₁-C₄-Alkyl, ein gesättigter oder ungesättigter aliphatischer Oxyrest mit 1 bis 4 C-Atomen, der gegebenenfalls durch C ₁-C₄-Alkoxy substituiert ist, CN oder NO₂ bedeutet, wobei R² ungleich Wasserstoff ist, wenn R¹ für n-Butyl steht,
**dadurch gekennzeichnet, daß** man eine Verbindung der Formel (IV) 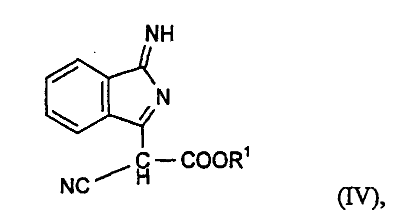 mit einem Benzimidazol der Formel (V) 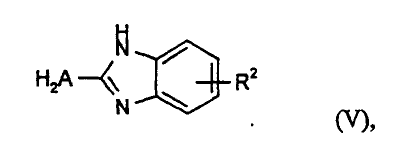 oder ein Aminoisoindolenin der Formel (VI) 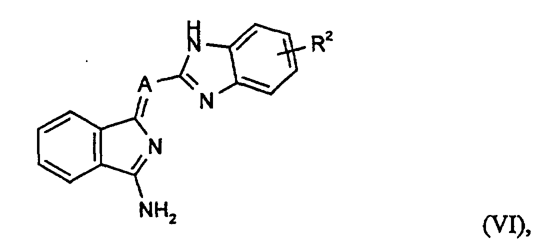 mit einem Cyanessigsäureester der Formel (VII)
NC-CH₂-COOR¹ (VII)
worin A, R¹ und R² die jeweils obengenannten Bedeutung besitzen, in Gegenwart einer organischen Säure kondensiert.

7. Verfahren gemäß Anspruch 6, **dadurch gekennzeichnet, daß** A für einen Cyanmethylenrest steht und.
R¹ für einen gesättigten oder ungesättigten, gegebenenfalls durch CN, oder gesättigten oder ungesättigten Oxyrest mit 1-4 C-Atomen, wie C₁-C₄-Alkoxy, Allyloxy und/oder ein Acyloxyrest substituierten aliphatischen geradkettigen Rest mit 4 bis 20 C-Atomen, steht, der gegebenenfalls durch einen oder mehrere Sauerstofatome unterbrochen ist und
R² Wasserstoff, Halogen, C₁-C₄-Alkyl, ein gesättigter oder ungesättigter aliphatischer Oxyrest mit 1 bis 4 C-Atomen, der gegebenenfalls durch C ₁-C₄-Alkoxy substituiert ist, CN oder NO₂ bedeutet

8. Verfahren gemäß Anspruch 6, **dadurch gekennzeichnet, daß** die Kondensation in Wasser oder einem wasserhaltigen Medium durchgeführt wird.

9. Verbindung der Formel (XV) oder deren tautomeren Formen 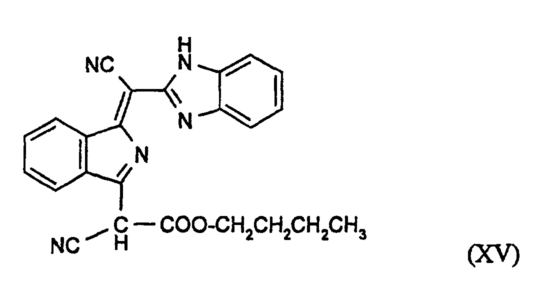 in Form ihrer α-, γ- und/oder ε-Kristallmodifikation, die durch folgende mittlere bis starke Reflexionen im Röntgenbeugungsdiagramm **gekennzeichnet** sind, wobei die Daten den Netzebenenabstand und die dazugehörigen relativen Intensitäten in Prozent (Zahlen in Klammern) bedeuten:
α-Modifikation: 9,298 (100); 19,300 (12); 23,080 (14); 24,199 (20); 24,574 (36); 26,457 (85); 27,795 (23); 28,656 (16)
γ-Modifikation: 5,007 (40); 5,474 (87); 6,316 (45,2); 8,393 (52); 10,045 (53); 16,646 (45); 25,772 (62); 26,377 (100)
ε-Modifikation: 5,034 (50); 6,113 (100); 7,070 (29); 7,328 (25); 10,603 (33); 12,263 (23); 12,767 (21); 24,889 (24); 26,263 (30); 26,682 (23).

10. Verwendung von Verbindungen der Ansprüche 1 oder 9 zum Färben und Bedrucken von vollsynthetischen oder halbsynthetischen hochmolekularen Stoffen.

11. Mischungen von einer oder mehreren Verbindungen der allgemeinen Formel I gemäß Anspruch 1, und einem oder mehreren Farbstoffen, wie sie üblicherweise zum Färben von Automobilbezugsstoffen verwendet werden.

12. Verwendung gemäß Anspruch 10, **dadurch gekennzeichnet, daß** Automobilbezugsstoffe gefärbt oder bedruckt werden.

## Claims

1. Compounds of the formula (I) 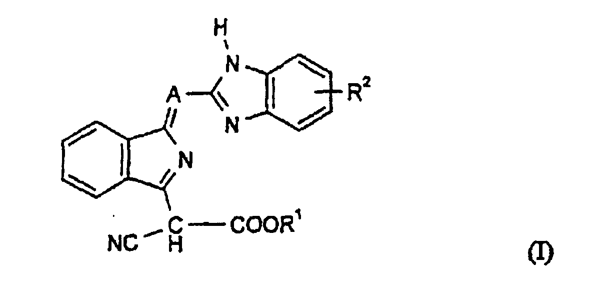 and tautomeric forms thereof, wherein
A represents N,
R¹ represents a saturated or unsaturated, optionally CN- or acyloxy-substituted aliphatic radical having 4 to 20 C atoms, or
A represents a cyanomethylene radical and
R¹ represents a saturated or unsaturated aliphatic straight-chain radical having 4 to 20 C atoms which is optionally substituted by CN, saturated or unsaturated oxyradical having 1-4 C atoms, such as C₁-C₄-alkoxy, allyloxy and/or an acyloxy radical which is optionally interrupted by one or mopre oxygen atoms and
R² denotes hydrogen, halogen, C₁-C₄-alkyl, a saturated or unsaturated aliphatic oxyradical having 1 to 4 C atoms, which is optionally substituted by C₁-C₄-alkoxy, CN or NO₂, R² not being hydrogen if R¹ represents n-butyl.

2. Compounds according to Claim 1, which correspond to the formula (II) 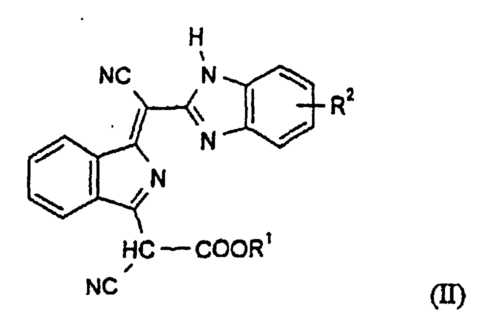 or tautomeric forms thereof, wherein
R¹ represents a saturated or unsaturated aliphatic straight-chain radical having 5 to 8 C atoms which is optionally substituted by CN, saturated or unsaturated oxyradical having 1-4 C atoms, such as C1-C4-alkoxy, allyoxy and/pr an acryloxy radical which is optionally interrupted by one or more oxygen atoms.

3. Compounds according to Claim 1 of the formula (I) which correspond to the formula (III) 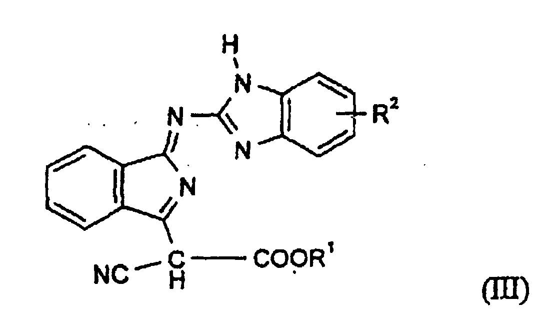 or tautomeric forms thereof, wherein
R¹ represents a saturated or unsaturated, optionally CN- or acyloxy-radical-substitued aliphatic radical having 4 to 20 C atoms.

4. Process for the preparation of compounds of the formula (I) according to Claim 1 or tautomeric forms thereof, **characterized in that** a compound of the formula (IV) 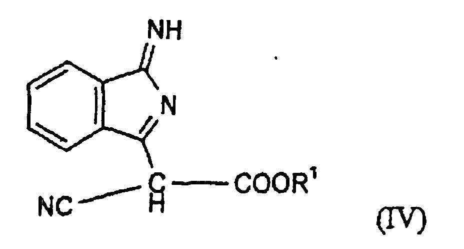 is subjected to a condensation reaction with a benzimidazole of the formula (V) 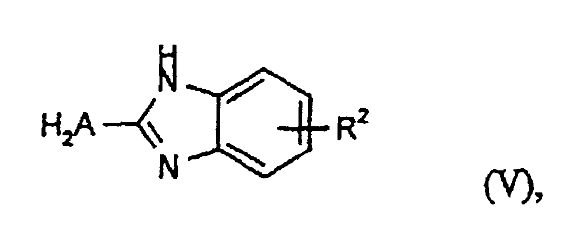 or an aminoisoindolenine of the formula (VI) 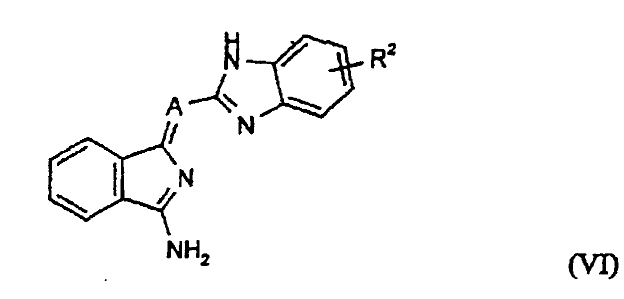 is subjected to a condensation reaction with a cyanoacetic acid ester of the formula (VII)
NC-CH₂-COOR¹ (VII)
wherein
R¹, R² and A have the meaning given in Claim 1.

5. Process according to Claim 4, **characterized in that**
A represents a cyanomethylene radical and
R¹ represents a saturated or unsaturated aliphatic straight-chain radical having 5 to 8 C atoms which is optionally substituted by CN, saturated or unsaturated oxyradical having 1-4 C atoms, such as C₁-C₄-alkoxy, allyloxy and/or an acyloxy radical which is optionally interrupted by one or more oxygen atoms and
R² denotes hydrogen, halogen, C₁-C₄-alkyl, a saturated or unsaturated aliphatic oxyradical having 1 to 4 C atoms, which is optionally substituted by C₁-C₄-alkoxy, CN or NO₂.

6. Process for the preparation of compounds of the formula (I) or tautomeric forms thereof 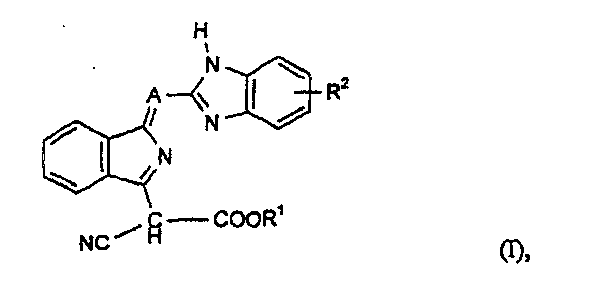 wherein
A represnts N,
R¹ represents a saturated or unsaturated, optionally CN- or acyloxy-substituted aliphatic radical having 4 to 20 C atoms,
A represents a cyanomethylene radical and
R¹ represents a saturated or unsaturated aliphatic straight-chain radical having 4 to 20 C atoms which is optionally substituted by CN, saturated or unsaturated oxyradical having 1-4 C atoms, such as C₁-C₄-alkoxy, allyloxy and/or an acyloxy radical which is optionally interrupted by one or more oxygen atoms and
R² denotes hydrogen, halogen, C₁-C₄-alkyl, a saturated or unsaturated aliphatic oxyradical having 1 to 4 C atoms, which is optionally C₁-C₄-alkoxy substituted, CN or NO₂, R² not being hydrogen if R¹ represents n-butyl,
**characterized in that** a compound of the formula (IV) 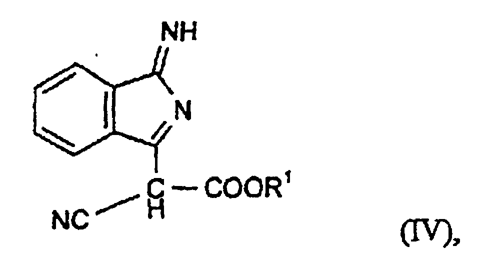 is subjected to a condensation reaction with a benzimidazole of the formula (V) 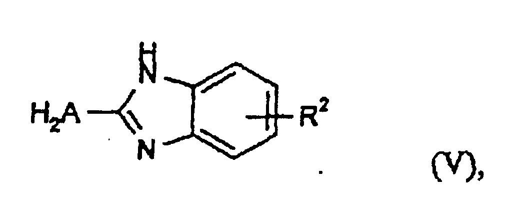 or an aminoisoindolenine of the formula (VI) 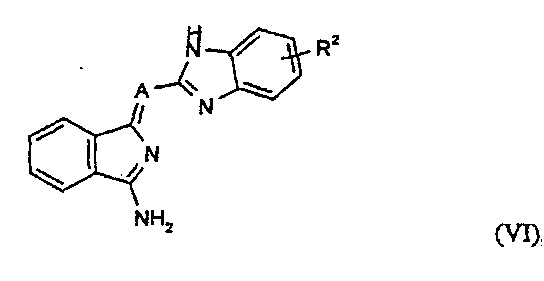 is subjected to a condensation reaction with a cyanoacetic acid ester of the formula (VII)
NC-CH₂-COOR¹ (VII)
wherein A, R¹ and R² have the particular abovementioned meaning,
in the presence of an organic acid.

7. Process according to Claim 6, **characterized in that** A represents a cyanomethylene radical and
R¹ represents a saturated or unsaturated aliphatic straight-chain radical having 4 to 20 C atoms which is optionally substituted by CN, saturated or unsaturated oxyradical having 1-4 C atoms, such as C₁-C₄-alkoxy, allyloxy and/or an acyloxy radical which is optionally interrupted by one or more oxygen atoms and
R² denotes hydrogen, halogen, C₁-C₄-alkyl, a saturated or unsaturated aliphatic oxyradical having 1 to 4 C atoms, which is optionally C₁-C₄-alkoxy substituted, CN or NO₂.

8. Process according to Claim 6, **characterized in that** the condensation is carried out in water or an aqueous medium.

9. Compound of the formula (XV) or tautomeric forms thereof 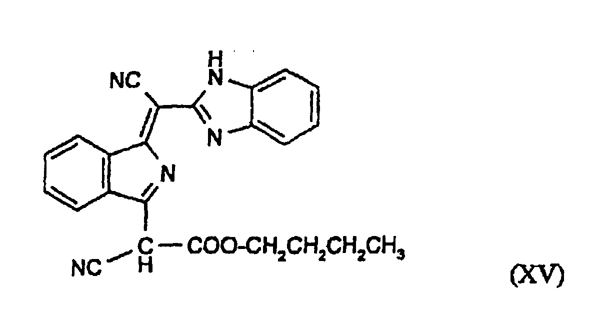 in the form of its α-, γ- and/or ε-crystal modification, which are **characterized by** the following moderate to intense reflections in the X-ray diffraction diagram, the data denoting the distance between lattice planes and the associated relative intensities in per cent (figures in parentheses):
α-modification: 9.298 (100); 19.300 (12); 23.080 (14); 24.199 (20); 24.54 (36); 26.457 (85); 27.795 (23); 28.656 (16)
γ-modification: 5.007 (40); 5.474 (87); 6.316 (45.2); 8.393 (52); 10.045 (53); 16.646 (45); 25.772 (62); 26.377 (100)
ε-modification: 5.034 (50); 6.113 (100); 7.070 (29); 7.328 (25); 10.603 (33); 12.263 (23); 12.767 (21); 24.889 (24); 26.263 (30); 26.682 (23).

10. Use of compounds of Claim 1 or 9 for dyeing and printing fully synthetic or semi-synthetic high molecular weight substances.

11. Mixtures of one or more compounds of the general formula I according to Claim 1 and one or more dyestuffs such as are usually used for dyeing automobile covering fabrics.

12. Use according to Claim 10, **characterized in that** automobile covering fabrics are dyed or printed.

## Revendications

1. Composés de formule (I) 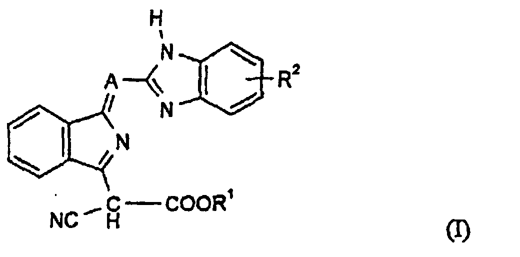 et leurs formes tautomères, formule dans laquelle
A représente N,
R¹ représente un radical aliphatique saturé ou insaturé, ayant de 4 à 20 atomes de carbone, éventuellement substitué par CN ou par un radical acyloxy, ou
A représente un radical cyanométhylène et
R¹ représente un radical aliphatique à chaîne droite, saturé ou insaturé, ayant de 4 à 20 atomes de carbone, éventuellement substitué par CN ou par un radical oxy saturé ou insaturé ayant de 1 à 4 atomes de carbone, tel qu'un radical alcoxy en C₁-C₄, allyloxy et/ou un radical acyloxy, qui est éventuellement interrompu par un ou plusieurs atomes d'oxygène et
R² représente un atome d'hydrogène ou d'halogène, un groupe alkyle en C₁-C₄, un radical oxy aliphatique saturé ou insaturé ayant de 1 à 4 atomes de carbone, qui est éventuellement substitué par un groupe alcoxy en C₁-C₄, ou représente CN ou NO₂, R² étant différent d'un atome d'hydrogène lorsque R¹ représente le groupe n-butyle.

2. Composés selon la revendication 1, qui correspondent à la formule (II) 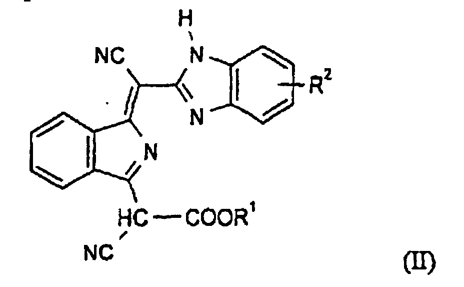 ou ses formes tautomères, dans laquelle
R¹ représente un radical aliphatique à chaîne droite, saturé ou insaturé, ayant de 5 à 8 atomes de carbone, éventuellement substitué par CN ou par un radical oxy saturé ou insaturé ayant de 1 à 4 atomes de carbone, tel qu'un radical alcoxy en C₁-C₄, allyloxy et/ou un radical acyloxy, qui est éventuellement interrompu par un ou plusieurs atomes d'oxygène.

3. Composés selon la revendication 1, de formule (I), qui correspondent à la formule (III) 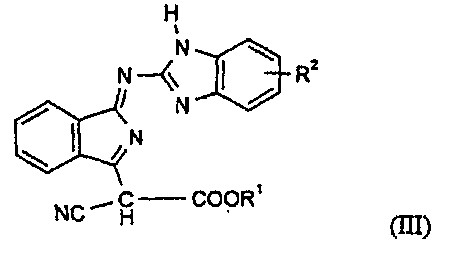 ou ses formes tautomères, dans laquelle
R¹ représente un radical aliphatique saturé ou insaturé, ayant de 4 à 20 atomes de carbone, éventuellement substitué par CN ou par un radical acyloxy.

4. Procédé pour la préparation de composés de formule (I) selon la revendication 1 ou de leurs formes tautomères, **caractérisé en ce qu'**on condense un composé de formule (IV) 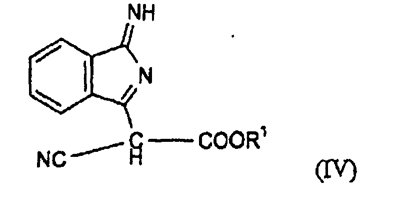 avec un benzimidazole de formule (V) 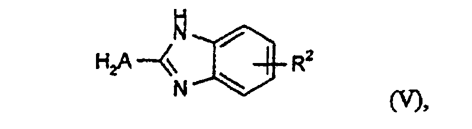 ou une amino-isoindolénine de formule (VI) 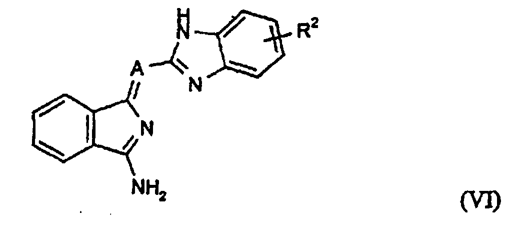 avec un ester d'acide cyanoacétique de formule (VII)
NC-CH₂-COOR¹ (VII)
R¹, R² et A ayant les significations indiquées dans la revendication 1.

5. Procédé selon la revendication 4, **caractérisé en ce que**
A représente un radical cyanométhylène et
R¹ représente un radical aliphatique à chaîne droite, saturé ou insaturé, ayant de 5 à 8 atomes de carbone, éventuellement substitué par CN ou par un radical oxy saturé ou insaturé ayant de 1 à 4 atomes de carbone, tel qu'un radical alcoxy en C₁-C₄, allyloxy et/ou un radical acyloxy, qui est éventuellement interrompu par un ou plusieurs atomes d'oxygène,
R² représente un atome d'hydrogène ou d'halogène, un groupe alkyle en C₁-C₄, un radical oxy aliphatique saturé ou insaturé ayant de 1 à 4 atomes de carbone, qui est éventuellement substitué par un groupe alcoxy en C₁-C₄, ou représente CN ou NO₂.

6. Procédé pour la préparation de composés de formule (I) ou de leurs formes tautomères, 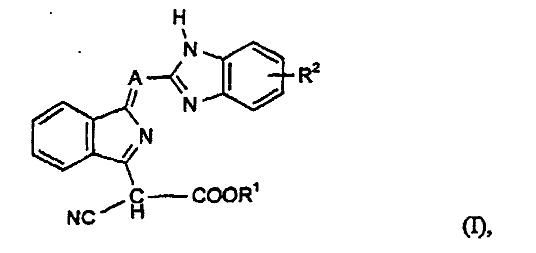 formule dans laquelle
A représente N,
R¹ représente un radical aliphatique saturé ou insaturé, ayant de 4 à 20 atomes de carbone, éventuellement substitué par CN ou par un radical acyloxy, ou
A représente un radical cyanométhylène et
R¹ représente un radical aliphatique à chaîne droite, saturé ou insaturé, ayant de 4 à 20 atomes de carbone, éventuellement substitué par CN ou par un radical oxy saturé ou insaturé ayant de 1 à 4 atomes de carbone, tel qu'un radical alcoxy en C₁-C₄, allyloxy et/ou un radical acyloxy, qui est éventuellement interrompu par un ou plusieurs atomes d'oxygène et
R² représente un atome d'hydrogène ou d'halogène, un groupe alkyle en C₁-C₄, un radical oxy aliphatique saturé ou insaturé ayant de 1 à 4 atomes de carbone, qui est éventuellement substitué par un groupe alcoxy en C₁-C₄, ou représente CN ou NO₂, R² étant différent d'un atome d'hydrogène lorsque R¹ représente le groupe n-butyle,
**caractérisé en ce qu'**on condense un composé de formule (IV) 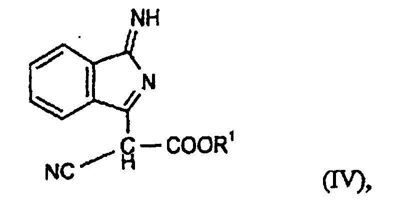 avec un benzimidazole de formule (V) 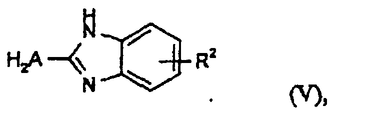 ou une amino-isoindolénine de formule (VI) 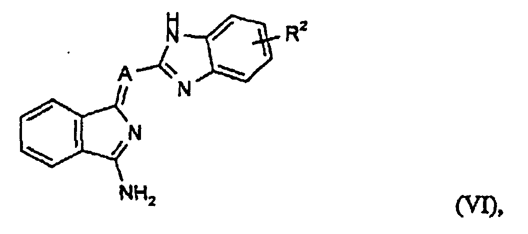 avec un ester d'acide cyanoacétique de formule (VII)
NC-CH₂-COOR¹ (VII)
A, R¹ et R² ayant chacun la signification donnée plus haut,
en présence d'un acide organique.

7. Procédé selon la revendication 6, **caractérisé en ce que** A représente un radical cyanométhylène et
R¹ représente un radical aliphatique à chaîne droite, saturé ou insaturé, ayant de 4 à 20 atomes de carbone, éventuellement substitué par CN ou par un radical oxy saturé ou insaturé ayant de 1 à 4 atomes de carbone, tel qu'un radical alcoxy en C₁-C₄, allyloxy et/ou un radical acyloxy, qui est éventuellement interrompu par un ou plusieurs atomes d'oxygène et
R² représente un atome d'hydrogène ou d'halogène, un groupe alkyle en C₁-C₄, un radical oxy aliphatique saturé ou insaturé ayant de 1 à 4 atomes de carbone, qui est éventuellement substitué par un groupe alcoxy en C₁-C₄, ou représente CN ou NO₂.

8. Procédé selon la revendication 6, **caractérisé en ce que** la condensation est effectuée dans l'eau ou un milieu contenant de l'eau.

9. Composé de formule (XV) ou ses formes tautomères 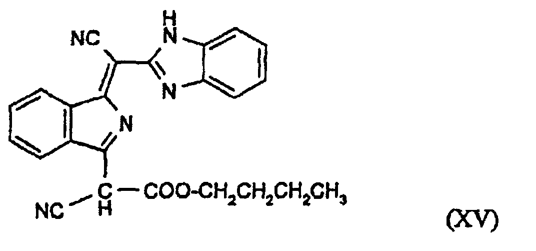 sous leurs formes cristallines α, γ et/ou ε qui sont **caractérisées par** les réflexions moyennes à fortes dans le diagramme de diffractions des rayons X, les données signifiant la distance interréticulaire et les intensités relatives correspondantes en pour cent (nombres entre parenthèses) ;
Forme α : 9,298 (100) ; 19,300 (12) ; 23,080 (14) ; 24,199 (20) ; 24,574 (36) ; 26,457 (85) ; 27,795 (23) ; 28,656 (16)
Forme γ : 5,007 (40) ; 5,474 (87) ; 6,316 (45,2) ; 8,393 (52) ; 10,045 (53) ; 16,646 (45) ; 25,772 (62) ; 26,377 (100)
Forme ε : 5,034 (50) ; 6,113 (100) ; 7,070 (29) ; 7,328 (25) ; 10,603 (33) ; 12,263 (23) ; 12,767 (21) ; 24,889 (24) ; 26,263 (30) ; 26,682 (23).

10. Utilisation des composés de la revendication 1 ou 9, pour la teinture et l'impression de matières de masse moléculaire élevée, semi-synthétiques ou totalement synthétiques.

11. Mélanges d'un ou de plusieurs composés de formule générale I selon la revendication 1, et d'un ou de plusieurs colorants tels qu'ils sont habituellement utilisés pour la teinture de matières concernant l'automobile.

12. Utilisation selon la revendication 10, **caractérisée en ce qu'**on teint ou imprime des matières concernant l'automobile.
